# EUROPEAN PATENT APPLICATION

(11) **EP 4 497 821 A1**
(43) Date of publication of application: **29.01.2025**
(21) Application number: 23382768.2
(22) Date of filing: 24.07.2023
(51) Int. Cl.: C12N 5/0783, A61K 39/00, C07K 14/725

(54) **METHOD FOR PRODUCING IMMUNE CELLS FOR IMMUNOTHERAPY**

(71) Applicant: Vivia Biotech, S.L., 28760 Tres Cantos, Madrid (ES)
(72) Inventor: Ballesteros, Joan, Tres Cantos, Madrid (ES); Primo Ramos, Daniel, Tres Cantos, Madrid (ES); Weng Jiang, Xian, Figueres (ES)
(74) Representative: Manuel Illescas y Asociados, S.L.

(57) **Abstract**

An ex *vivo* method for producing immune cells for immunotherapy, wherein the method comprises activating and/or expanding immune cells in a sample comprising said immune cells, comprising: (a) adding a cell culture medium and culturing agents to the sample, thereby obtaining a cell culture; (b) culturing the cell culture obtained in step (a), wherein in this step the immune cells are cultured with said culturing agents and with live cancer cells, wherein said live cancer cells were already present in the sample and/or were added in step (a) to the sample; and (c) enriching the fraction of immune cells in the cell culture obtained in step (b). Immune cells include T cells, NK cells, and engineered immune cells, such as chimeric antigen receptor T cells (CAR-T), T cell receptor T cells (TCR-T), chimeric antigen receptor NK cells (CAR-NK), and T cell receptor NK cells (TCR-NK).

## Description

### TECHNICAL FIELD

The present invention relates to the technical field of methods for producing T cells or NK cells for immunotherapy. In particular, the present invention relates to methods for producing T cells or NK cells for immunotherapy in which T cells or NK cells are incubated with live cancer cells.

### BACKGROUND ART

There are several types of T cell therapies. In one approach, endogenous patient T cells are used, such as tissue-infiltrating lymphocytes (TILs). TILs express surface receptors that specifically target cancer antigens and exhibit cancer-selective cytotoxicity. Manufacturing of TILs is commonly performed activating T cells with activators such as CD3/CD28 beads and expanding them for 2-3 weeks with IL-2 cytokine, sometimes supplemented with IL-2, IL-15, IL-21, and IL-7. In this process, tumor cells die within few days and most of the expansion days are performed with only T cells present. An important problem of these TIL therapies is that they lose their cancer-killing activity during the expansion.

In other approach, genetically engineered T cells are used, such as chimeric antigen receptor T cells (CAR-T). CAR-Ts are genetically modified T cells that can recognize specific moieties on target tumor cells. Several additional constructs can be engineered along the CAR constructs, such as immune check point inhibitors. Although CAR T cell therapy has significant anticancer benefits, its delivery presents a key limitation. The complexity of the technology requires it to be administered at specialized centers, often leading to treatment delays. In the interim, bridging chemotherapy is typically utilized, a process that spans 28 days. Unfortunately, this bridging therapy accounts for a significant portion of treatment-related fatalities.

According to a further approach, a TCR receptor with the desired antigen selectivity is engineered into a T cell. A particular example is neoantigen TCRs that recognize an antigen expressed only on tumor cells. These T cell therapies share the problems of CAR T therapies in terms of complicated centralized logistics, long manufacturing lead times, and the need for bridging chemotherapy, responsible for the majority of treatment induced deaths.

Moreover, conventional manufacturing methods for T cell therapies have steered clear of incubating T cells with live tumor cells due to the concern that residual live tumor cells could end up in the final T cell therapy product, potentially posing a risk to the patient.

A major problem for manufacturing of cell therapies is that T cells lose cancer-killing potential when they are expanded *ex vivo.* This has been shown clinically from analyzing patients treated with the first approved TIL therapy by lovance Biotherapeutics Inc. originally from Prof. Rosenberg at the National Institutes of Health, U.S.A.. The method consisted in extracting TILs from the melanoma tumor mass, activated with CD3/CD28 beads and expanded with 500 lU/ml of IL-2. The patient responses from this TIL therapy have been analyzed and TIL expansion duration has shown to be a statistically significant predictor of clinical response in melanoma (Besser et al. (2020)).

The reason for the loss of TIL activity during expansion is that the tumor-specific antigens (TSA) T cells are progressively lost over time. According to the prior art, standard manufacturing methods to expand TILs preferentially expands non-tumor related T cells, while TSA T are overgrown by non-tumor related T cells due to slower growth.

Current T cell therapies when approved reach very few patients worldwide due to their complicated centralized manufacturing logistics and high costs. As an example, approximately 50% of patients referred for approved CAR T therapies cannot receive the treatment due to rapidly progressive disease patients (28% cannot wait weeks for treatment administration) (Spanjaart et al. (2022)).

Existing T cell therapies necessitate a manufacturing period of 2-3 weeks, during which patients typically undergo a 28-day cycle of bridging chemotherapy. This interim treatment is the primary contributor to toxicity and yields an estimated treatment-related mortality rate of 20-30%. Because all prior art cell therapies require this chemotherapy cycle, this major problem is not commonly highlighted.

Another type of immune cell therapy is NK cells. These cells represent the innate immune system. NK Cell therapy is being developed for multiple cancers. There are also cell therapies in development including genetic engineering of chimeric antigen receptor NK cells (CAR-NK), and engineered T cell receptor NK cells (TCR-NK), and/or other genes, similar to T cell therapy. Similar to T cells, manufacturing NK cell therapies, including genetically engineered NK therapies, can benefit from activation and/or expansion including live tumor cells, autologous or allogenic. Tumor cells provide on their surface the distress ligands recognized by NK receptors such as NKG2D receptors, that recognizes 8 ligands in the tumor cell surface. Activation/expansion of NK cell therapy and their manufacturing have many parallels with T cell therapies, including conditions and reagents used.

Several types of NK cell therapies have been explored for the treatment of blood cancers, including autologous, allogeneic, CAR-NK, NK Cell Line, NK cell engagers (equivalent of T cell engagers).

For autologous methods there's usually a purification process to obtain NK cells that can be either apheresis, gradient centrifugation (Ficoll), magnetic assisted cell sorting (MACS), fluorescence assisted cell sorting (FACS).

There are different methods to activate NK cells:
1. Cytokine Activation: One of the most common methods for activating and expanding NK cells involves using cytokines, which are small proteins that modulate the immune response. Interleukin-2 (IL-2) and Interleukin-15 (IL-15) are two cytokines frequently used for this purpose. They can stimulate NK cell proliferation and enhance their cytotoxicity. IL-21, IL-12, IL-18 are also used sometimes. However, there's a risk of activating other immune cells with these cytokines that can lead to an overactive immune response and potential side effects.
2. Feeder Cells: Another method of expanding NK cells involves the use of feeder cells, which are cells that have been genetically modified to express membrane-bound IL-21 and other ligands for activating receptors on NK cells. Examples include K562-mb21-41BBL feeder cells. The feeder cells provide a continuous source of stimulatory signals, promoting NK cell activation and expansion. These cells are irradiated and thus not alive. The downside of this approach is the complexity of the procedure and the potential risk of feeder cells contaminating the NK cell product.
3. Artificial Antigen-Presenting Cells (aAPCs): These are cells that are engineered to present specific antigens along with co-stimulatory signals to NK cells, which helps to activate and expand the NK cells. This technique can be customized to target specific types of cancer cells. These cells are irradiated and thus not alive.
4. Genetic Modification: Genetic modification of NK cells can not only equip them with specific receptors to target cancer cells but also can be used to enhance their proliferation and survival. CAR-NK cells are one example of this approach.
5. Pharmacological Agents: Certain drugs can also be used to activate and expand NK cells. Lenalidomide and bortezomib, for instance, are drugs that have been found to enhance NK cell activation.

Most common protocol for the activation and expansion of autologous NK cells involves the use of cytokines like IL-2. The steps are: 1) Isolation of PBMCs, 2) Isolation of NK cells from PBMCs, 3) Expansion and activation of NK cells, 4) Quality Control (CD56 and/or CD16).

The reagent cocktails used in the prior art for activation of NK cells are IL-2, I-15, IL-21 and combinations thereof, and potential use of irradiated feeder cells.

There is therefore a need to improve manufacturing methods of T cells and NK cells for immunotherapy.

### SUMMARY OF INVENTION

The terms "tumor-specific antigens (TSA) T cells" and "tumor-associated antigens (TAA) T cells" refer to T cells whose T Cell Receptor (TCR) recognizes tumor-specific or tumor-associated antigens, respectively. Tumor specific antigens refers to antigens derived only from the tumor, while tumor-associated antigens refer to antigens derived from the tumor and also from other tissues or sources. These TSA T cells can be identified by Peptide Presenting MHC Complex, small proteins mimicking the antigen-MHC complexes in the surface of tumor cells, commonly grouped adding multiple units per molecule in e.g. tetramers (4), pentamers (5), or dextramers (10), detected by fluorochromes or Next Generation Sequencing of DNA tags.

The term "tumor-infiltrated lymphocytes (TILs)" refers to T cells present in or adjacent to the tumor, which are more enriched in TSA/TAA T Cells.

The term "CD3/CD28 beads" refer to magnetic beads coated with a mixture of monoclonal antibodies against the CD3 and CD28 cell surface molecules of human T cells. The beads include, but are not limited to, magnetic polystyrene beads.

The term "CD3" refers to cluster of differentiation 3. It is a protein expressed on T cells.

The term "CD28" refers to cluster of differentiation 28. It is a protein expressed on T cells.

The term "CD123" refers to the interleukin-3 receptor.

The term "HLA" refers to "human leukocyte antigen". HLA are cell-surface proteins responsible for regulation of the immune system.

The term "PD-1" refers to "programmed cell death protein 1", an immune checkpoint.

The term "TIM-3" refers to "T-cell immunoglobulin and mucin-domain containing-3", an immune checkpoint.

The term "LAG-3" refers to "lymphocyte-activation gene 3", an immune checkpoint.

The term "CTLA-4" refers to "cytotoxic T-lymphocyte-associated protein 4", an immune checkpoint.

The term "TIGIT" refers to "T cell immunoreceptor with Ig and ITIM domains", an immune checkpoint.

The term "NKG2A" refers to type A of NKG2, a receptor for natural killer cells (NK cells).

The term "IDO1" refers "indoleamine 2, 3-dioxygenase 1".

The term "cytokines" refers to small proteins important in cell signaling. Cytokines promote T cell proliferation during manufacturing. Examples include, but are not limited to, IL-2, IL-15, IL-21, and IL-7.

A major problem for manufacturing of cell therapies is that they lose the principal cancer-killing T cells, the TSA/TAA T cells when the cells are expanded *ex vivo.* Therefore, the problem to be solved is to improve manufacturing methods of T cells for immunotherapy which avoids the loss of TSA/TAA T cells.

The present invention solves this problem by activating and expanding tumor-reactive T cells with live tumor cells. Live tumor cells provide a stimulus to cancer-killing immune cells such as antigen presentation and facilitates activation and expansion of cancer-killing immune cells.

Prior art cell therapies require 2-3 weeks of manufacturing time which requires treating patients with a bridging chemotherapy cycle, which is the major source of toxicity and treatment-induced mortality. Therefore, an additional problem to be solved is to accelerate manufacturing methods, to avoid bridging chemotherapy for patients.

The present invention solves this problem by shortening the manufacturing process to only 7 days to avoid the bridging chemotherapy cycle for patients.

The present invention provides an *ex vivo* method for producing immune cells for immunotherapy, wherein the method comprises activating and/or expanding immune cells in a sample comprising said immune cells, the method comprising the following steps:
(a) adding a cell culture medium and culturing agents to the sample, thereby obtaining a cell culture;
(b) culturing the cell culture obtained in step (a), wherein in this step the immune cells are cultured with said culturing agents and with live cancer cells, wherein said live cancer cells were already present in the sample and/or were added in step (a) to the sample; and
(c) enriching the fraction of immune cells in the cell culture obtained in step (b).

In the method of the present invention, immune cells are incubated with live tumor cells, which promotes expansion of tumor-reactive immune cells. Live tumor cells provide a stimulus to cancer-killing immune cells such as antigen presentation and facilitates activation and expansion of immune cells.

The method of the present invention can be applied to T cell therapies and NK cell therapies. NK cell therapies include therapies based on NK cells, engineered chimeric antigen receptor NK cells (CAR-NK), and engineered T cell receptor NK cells (TCR-NK). Example 11 shows activation and expansion of NK cells.

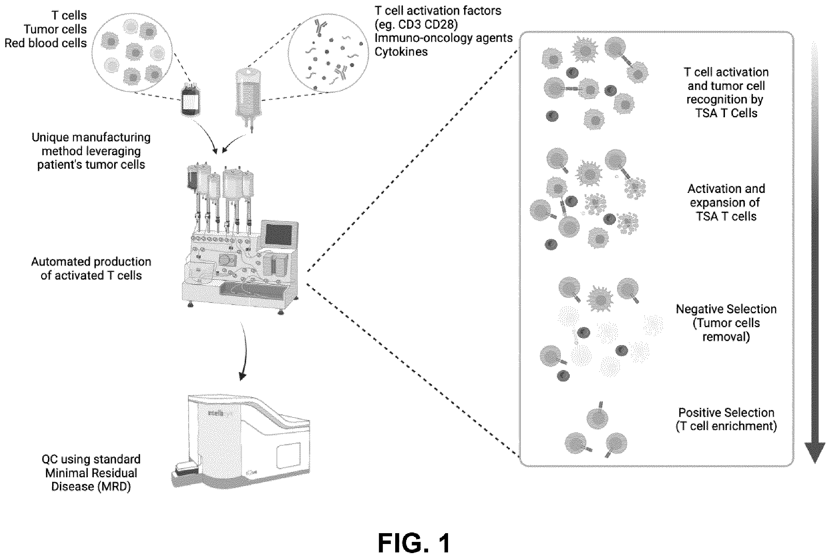

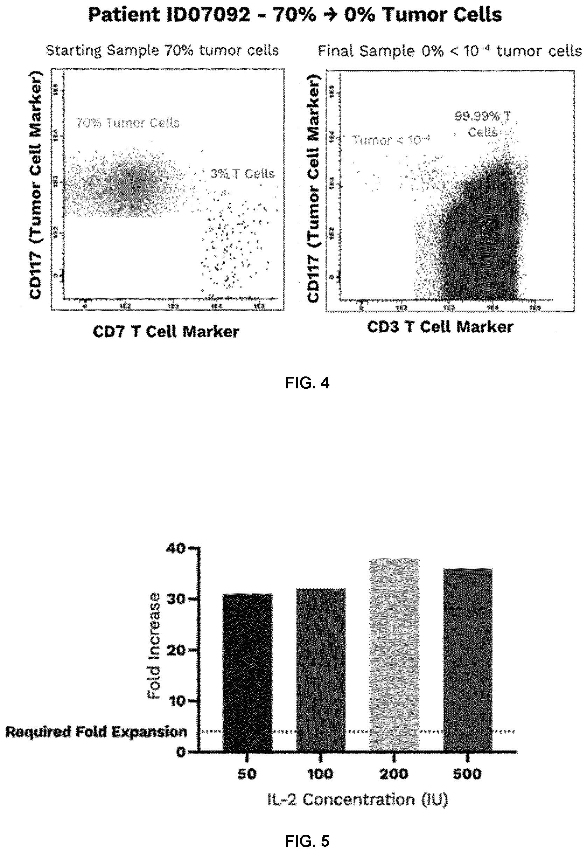

In a preferred embodiment, T cells are expanded by incubating with its autologous tumor cells, which naturally promote recognition and expansion of tumor-reactive T cells. **Fig. 1** shows a scheme of our proposed manufacturing process. The patient sample, for example a bone marrow sample from a patient suffering AML (Acute Myeloid Leukemia), is extracted and incorporated to an automated cell manufacturing system such as a CliniMacs Prodigy shown left in the middle panel. Sample can be inserted with all its components such as red blood cells etc..., or isolated T cells, or in a case of a solid tumor disaggregating the cells first prior to insertion into the automated system. A cocktail of factors for the manufacturing process is also inserted, shown to the right of the sample. This cocktail includes factors for T cell activation such as CD3 beads, CD28 beads, cytokines to facilitate T cell proliferation such as IL2, potentially adding also IL15, IL21, IL7, plus potentially other immune-oncology factors such as immune check point inhibitors and other immunomodulating agents. In a preferred embodiment, all these factors in the cocktail are added to the sample from the beginning and all together at once. In another embodiments, some of these factors are not added or are added at different times. All manufacturing steps can be performed in the automated system. Red blood cell removal can be performed preferably when the media is exchanged at day 5, but could also be performed at the beginning or after the activation and expansion is complete. In addition to the 2 manufacturing steps using an automated system, there are some steps that occur within the automated system. Inside the automated system, 4 phases are distinguished, in addition to the red blood cell removal, shown on the right side of the figure 4: 1) T cell activation, including activation by recognition of tumor-presented antigens by TSA T cells. 2) Activation and expansion of TSA T cells and other T cells. 3) To complete the manufacturing, it is needed to remove potentially remaining tumor cells, and this is done by a negative selection using magnetic beads coated with selective molecules that recognize tumor cell surface markers such as antibodies. 4) The T cells can be positively selected using magnetic beads coated with CD4 and CD8 targeting molecules such as antibodies, a standard method to purify T cells for T cell therapy. These two bead isolation steps are an innovation never performed in T cell manufacturing. Once the TIL therapy has been manufactured inside the automated system, and prior to administration to a patient, there should be some Quality Control (QC). The proposed QC would be to measure viability of the T cells (standard measurement for T cell therapies), and also a special QC to make sure there are no tumor cells left that could be administered to the patient. A Quality Control (QC) to ensure the absence of tumor cells in the final T cell product will be a clinical standard Minimal Residual Disease (MRD) test, when available for a cancer type or an analogous method for other cancers, that quantifies traces of tumor cells left in a patient sample suffering from a hematological malignancy. This MRD test serves as a quality control measure to ensure the absence of the patient's tumor cells, addressing a similar safety concern in autologous stem cell transplantation for hematological malignancies. In all hospitals treating patients with such malignancies, including AML, this has become a routine practice to assess treatment efficacy. The standard criterion is that no tumor cells should be detectable at a sensitivity limit of 0.01%, determined by flow cytometry. This criterion is more restrictive than one the applied by European Leukemia Network (ELN) guideline for considering MRD negative. MRD is commonly used at hospitals to track AML patients in Complete Remission after treatment to measure the small % of tumor cells left, as a prognostic factor of potential time to relapse. It is also used after allogenic hematopoietic stem cell transplant to confirm the absence of cancer cells. It is a validated diagnostic assay to measure of trace amounts of tumor cells left in AML patients, and it can be applied to measure trace amounts of tumor cells left in the TIL final product prior patient administration Example 2 describes that this MRD negative threshold for T cells from 8 AML samples manufactured according to the method of the present invention has been achieved.

In a preferred embodiment, the method of the present invention has very simplified logistics. Manufacturing requires only 2 steps plus a QC. First step is to extract the tumor sample from the patient with its TILs, a work routinely performed at the hospital. Then, the sample is introduced into the automated cell therapy system (e.g. CliniMacs Prodigy). It is proposed to insert the crude sample, just as it is extracted, without any manipulation. For TIL and CAR T therapies it is common to perform procedures such as an apheresis to separate the T cells from the rest of the cells in the sample, and it is proposes to avoid this manipulation step. The whole bone marrow sample is inserted into the CliniMacs Prodigy will be diluted with PBS, our cocktail will be added to the media, and incubation will start. A media exchange would be necessary, preferably at days 3, 4, or 5. A major issue is the red blood cells, because in a bone marrow sample >99% of cells may be red blood cells. For example, the CliniMacs Prodigy has the means to remove most of these red blood cells. This removal is performed ideally when the media is exchanged at days 3-4-5. For AML as shown in the examples, at day 5 typically all TILs are activated and all tumor cells have been killed. In days 6 and 7 it is observed the fastest expansion to achieve the desired activated TIL numbers for patient administration.

On day 7 a small sample is extracted for QC, composed of viability and Minimal Residual Disease (MRD) measurements by flow cytometry. It is common to measure MRD also by Next Generation Sequencing, although flow cytometry is faster to avoid delays in patient treatment. Additional QCs may be added. If the activated TILs extracted have poor viability or MRD positive the product would be rejected for clinical use. If the QC is satisfactory, the activated TILs are harvested and administered to the patient. Overall, the manufacturing process can be accomplished in only 2 steps, followed by a QC check. This simplified logistics is very important to enable decentralized manufacturing, where the automated system such as the CliniMacs Prodigy can be operated in each hospital under regulated conditions. This enables to use always fresh samples and cells without any negative effects from cryopreservation. The result is a 7 day vein-to-vein time at the point the care.

In a preferred embodiment, a washing step removes all reagents from our cocktail before administration to the patient, since these reagents include mouse or rabbit antibodies that may cause an antigenic reaction.

It is proposed to manufacture T cell at point-of-care, in each hospital, using an automated cell therapy system, for example the CliniMacs Prodigy but not limited to, the gold standard and already approved for T cell manufacturing. The process for AML is as follows:

### Day 1

A 30-50 ml of bone marrow sample from the AML patient is extracted. For 30-50 ml a complete sedation is not needed and can be extracted following standard hospital procedures.

In a CliniMacs Prodigy automated system, the sample is incubated by adding:
(a) The AML sample, without any processing, including plasma, red blood cells, platelets, etc. This simplifies sample handling to a minimum.
(b) Cocktail, including the agents described above, all purchased from commercial sources:
   (i) Activation factors, for example CD3/CD28 beads, or CD3xCD123, or CD3xHLA, or others
   (ii) anti-PD-1 + anti-TIM-3 + anti-LAG-3 + anti-CTLA-4 + anti-TIGIT + anti-NKG2A
   (iii) IDO1 inhibitor, Adenosine 2A receptor antagonist
   (iv) IL-2 50 IU/ml + IL-15 + IL-21

### Day 5

(a) Red blood cells removal inside an automated cell therapy manufacturing device.
(b) Change of media within the automated cell therapy manufacturing device providing new nutrients, adding again the cocktail added in Day 1 (see (b) above). Adding JQ1 (last 2 days only at low dose)
(c) Potentially transduce or transfect activated T cells with CAR or TCR construct. All inside the automated cell therapy manufacturing device. Depending on the specific CAR/TCR to be engineered in a specific cancer type, instead of Day 5 it may be needed to use day 7 extending manufacturing for 1 or 2 more days. Alternatively, the CAR/TCR can be transduced earlier, at days 1-2-3-4.

### Day 7

Process is the same for TIL or CAR/TCR TIL products. For CAR/TCR-TIL products after the transduction or transfection process, remaining vectors are washed away and administer the cell therapy products to the patient where stable integration of the CAR/TCR DNA into the TIL genome may occur inside the patient body, following the current trend within fast manufacturing CAR-T.
(a) Double isolation T cells using magnetic beads first to remove remaining AML cells left and second to select T cells, all inside a suitable cell therapy manufacturing device.
(b) Wash T cells to remove ancillary reagents.
(c) Small aliquot final T cell product produced for QC in a flow cytometry comprising:
   (i) MRD to ensure absence of tumor cells with 0.01% (10⁻⁴) detection limit.
   (ii) Viability cells >90%
(d) If QC is positive, then final product T therapy delivered for patient infusion.

In embodiments, the method of the present invention further comprises, after step (b):
(i) removing cancer cells from the cell culture obtained in step (b).

In the previous embodiment, said step (i) is carried out by a technique selected from the group consisting of: magnetic beads, FACS sorting, and affinity chromatography.

In a preferred embodiment of the method of the present invention, said step (i) removes live and dead cancer cells. In other preferred embodiment of the method of the present invention, said step (i) removes only live cancer cells.

In embodiments of the method of the present invention, magnetic beads recognizing cell surface markers of the cancer cells are used. In a preferred embodiment, said magnetic beads recognize a cell surface marker selected from the group consisting of: CD33, CD123 and CD117.

In embodiments of the method of the present invention, removing live and dead cancer cells from the cell culture obtained in step (i) comprises:
(i) adding magnetic beads coated with molecules recognizing cell surface markers of the cancer cells; and
(ii) removing the fraction comprising the magnetic beads, thereby removing the cancer cells bound to the magnetic beads from the cell culture.

In embodiments of the method of the present invention, wherein step (c) is carried out by a technique selected from the group consisting of: magnetic beads, FACS sorting, and affinity chromatography.

In embodiments of the method of the present invention, magnetic beads recognizing cell surface markers of the immune cells are used. In a preferred embodiment, said magnetic beads recognize a cell surface marker selected from CD4 and CD8.

In embodiments of the method of the present invention, enriching the fraction of immune cells in the cell culture obtained in step (b) comprises:
(i) adding magnetic beads coated with molecules recognizing cell surface markers of the immune cells; and
(ii) selecting the fraction of the cell culture comprising the magnetic beads, thereby enriching the fraction of immune cells in the cell culture obtained from the previous sub step (i) and removing the remainder fraction of the cell culture.

In embodiments of the method of the present invention, the immune cells are selected from the group consisting of: tumor infiltrating lymphocytes (TIL), tumor-specific antigens (TSA) T cells, neoantigen T cells, tumor-associated antigens (TAA) T cells, engineered chimeric antigen receptor T cells (CAR-T), engineered T cell receptor T cells (TCR-T), NK cells, engineered chimeric antigen receptor NK cells (CAR-NK), and engineered T cell receptor NK cells (TCR-NK). In a preferred embodiment of the method of the present invention, tumor-associated antigens (TAA) T cells includes subsets such as peptide presenting MHC complex-positive TSA T cells.

In embodiments of the method of the present invention, the culturing agents are selected from the group consisting of: immune cell activators, immune checkpoint inhibitors, immune modulatory agents, and cytokines.

In a preferred embodiment of the method of the present invention, immune cell activators are selected from mixture of CD3/CD28 beads and bispecific antibodies, CD3/CD28 beads and a mixture of anti-CD3 and anti-CD28 monoclonal antibodies.

In embodiments of the method of the present invention, the cell culture medium comprises human serum. Adding human serum to the cell culture medium enhances T cell expansion.

Incubating immune cells with live tumor cells is improved by adding culturing agents that facilitate activation/expansion, such as immune cells activators, immune checkpoint inhibitors, immune modulatory agents, and cytokines.

Immune checkpoint inhibitors (ICI) facilitate reactivation of TSA T cells. Examples of immune checkpoints include, but are not limited to, PD-1, LAG-3, TIM-3, NKG2A, CTLA-4, and TIGIT. Immune checkpoint inhibitors include agents recognizing any of said immune checkpoints such as antibodies.

Immune modulatory agents further facilitate reactivation and expansion of TSA T cells by removing immunosuppressive mechanisms. Examples of immune modulatory agents include, but are not limited to, IDO1 inhibitors, JQ1 and adenosine 2A receptor antagonists.

Cytokines promote T cell proliferation during manufacturing. Examples include, but are not limited to, IL-2, IL-15, IL-21, and IL-7.

In embodiments of the method of the present invention, the culturing agents are selected from the group consisting of: CD3/CD28 beads, bispecific antibody, anti-PD-1, anti-TIM-3, anti-LAG-3, anti-CTLA-4, anti-TIGIT, anti-NKG2A, IDO1 inhibitor, adenosine 2A receptor antagonist, IL-2, IL-15, IL-21, and JQ1.

In a preferred embodiment of the method of the present invention, the concentration of IL-2 is 5- 50 IU/ml.

The standard concentration of IL-2 is 500 IU/ml. The concentration of IL-2 of 50 IU/ml facilitates T cell proliferation, so that non-TSA T cells are not strongly promoted to proliferate which could overgrow TSA T cells.

In embodiments of the method of the present invention, the bispecific antibody is selected from CD3/CD123 and CD3/HLA.

In embodiments of the method of the present invention, the sample is selected from the group consisting of: tumoral sample, bone marrow, whole bone marrow, blood, whole blood, and peripheral blood.

In embodiments of the method of the present invention, the sample is from a subject having a cancer.

The sample used in the method of the present invention can proceed from a subject having multiple cancer types. For solid tumors, fragmentation and disaggregate the tumor mass may be implemented. The patient sample from some solid tumors may have an insufficient number of tumor cells for this manufacturing process, and in this case, the incubation can be supplemented with live cancer cell lines for the cancer type of the patient TILs.

The sample used in the method of the present invention can proceed from the bone marrow sample from a solid tumor patient. Bone marrow samples from solid tumor patients are a reservoir of TSA/TAA T cells that can be reactivated and used for autologous cell therapy. In this case, the incubation can be supplemented with live cancer cells, either cancer cells from the same patient tumor mass, or cell lines for the cancer type of the patient TILs.

In embodiments, the method of the present invention further comprises, after step (b), genetically engineering the immune cells for obtaining engineered immune cells selected from the group consisting of: engineered chimeric antigen receptor T cells (CAR-T), engineered T cell receptor T cells (TCR-T), engineered chimeric antigen receptor NK cells (CAR-NK), and engineered T cell receptor NK cells (TCR-NK).

In embodiments, the method of the present invention further comprises genetically engineering the immune cells for obtaining engineered immune cells at any of the following days from the start of the method: day 1, day 2, day 3 and day 4.

According to this embodiment, the method of the present invention provides genetically engineered chimeric antigen receptor, T cell receptor and/or other constructs on T cells or NK cells for enhanced activity.

In a preferred embodiment, CAR and/or TCR constructs are genetically engineered on TILs activated and expanded incubating with live tumor cells, rather than the standard peripheral blood T cells. The CAR and/or TCR constructs and/or other constructs are transduced or transfected into TILs, thereby generating CAR-TIL or TCR-TIL constructs. Genetic engineering of T cells has evolved with many different approaches enhancing both activity and safety, that a CAR-TIL or TCR-TIL constructs can leverage for an enhanced cell therapy product. There have been several proposals to use TILs to manufacture CAR and TCR T cell therapies, but the problem in these proposals is that the manufacturing method of TILs required long 2-3 weeks expansions in which they lose a substantial % of their TSA/TAA T cells and thus their cancer-killing activity. In the method of the present invention in which TILs are incubated with live tumor cells prevents this problem. Activated TILs enriched in TSA/TAA TILs serve as a better source to transduce/transfect CAR, TCR, or other constructs. Advantages of using TILs rather than peripheral blood (PB) T cells for manufacturing of CAR or TCR T cells are:
- TSA/TAA TILs can recognize and kill cancer cells while PB T cells don't. This may be especially important for the known resistance mechanisms of some cancer cells not expressing the target of the engineered CAR or TCR. Antigen downregulation and early chimeric antigen receptor (CAR) T-cell loss have emerged as two major challenges threatening outcomes following CD19-specific CAR-T cell therapy. Patients treated with CD19 CAR Ts may relapse with CD19 negative clones, that the CAR-T CD19 cannot recognize, but the TILs would recognize and kill as it reappears in small numbers since the TSA/TAA T cells will be functional.
- Enhanced tumor accumulation: TILs and specially tumor-reactive TILs such as TSA/TAA are able to migrate towards the tumor site, supposedly following a chemokine gradient. PB T cells on the contrary lack this capacity, and kinetic studies of CAR on PB T cells in vivo distribution show a wide distribution across tissues in the first days, with many CAR-T cells dying, until some CAR-T cells find the tumor sites and they expand locally killing effectively the tumor. Thus CAR-TILs or TCR-TILs may migrate faster and in larger numbers to tumor sites vs PB CAR/TCR T cells and may kill as effectively once at the tumor site, resulting in faster therapeutic benefit to patients. CAR-TILs and TCR-TILs may also have enhanced viability vs CAR-T and TCR-T cells in vivo accumulating at larger numbers *in vivo.*

There exist particular scenarios where peripheral blood (PB) T cells may not serve as an optimal source for CAR/TCR engineering, especially in cases of Acute Myeloid Leukemia (AML). For AML patients, the bone marrow provides the primary tissue for leukemic cells, and PB in these patients is often densely infiltrated with tumor cells, generating a challenge for T cell therapies such as CAR-T. Consequently, the T cell count in PB can significantly drop, sometimes falling below 50% of the standard T cell count. Moreover, the functionality of AML PB T cells with regards to activation, expansion, and transduction or transfection of the CAR construct is compromised, suggesting a pathological impact. Our invention proposes the use of bone marrow TILs as an alternative to PB T cells, addressing this central issue in manufacturing AML T cell therapies such as CAR-T and TCR-T. Our method not only efficiently activates and expands bone marrow T cells, but also generates an adequate number of T cells for therapies such as CAR T or TCR-Ts, as demonstrated in Example 2.

In embodiments of the method of the present invention, step (b) comprises culturing the cell culture for 7 days or less.

In embodiments of the method of the present invention, step (b) comprises the following sub steps:
(b.i) culturing the cell culture obtained in step (a) for 5 days or less; and
(b.ii) changing the cell culture medium of the cell culture obtained in sub step (i), further adding culturing agents, and culturing the cell culture for at least 2 days.

In the previous embodiment, after step (b.i), red blood cells are depleted.

In the previous embodiment, in sub step (b.ii), the culturing agents comprises IL-2, IL-15, and IL-21 and JQ1.

In a preferred embodiment, in sub step (b.ii), the cell culture is cultured for 2-9 days.

In embodiments of the method of the present invention, step (b) comprises the following sub steps:
(b.i) culturing the cell culture obtained in step (a) for 5 days or less;
(b.ii) changing the cell culture medium of the cell culture obtained in sub step (i), further adding culturing agents, and culturing the cell culture for at least 2 days; and
(b.iii) genetically engineering the immune cells for obtaining engineered immune cells selected from the group consisting of: engineered chimeric antigen receptor T cells (CAR-T), engineered T cell receptor T cells (TCR-T), engineered chimeric antigen receptor NK cells (CAR-NK), and engineered T cell receptor NK cells (TCR-NK).

In the previous embodiment, after step (b.i), red blood cells are depleted.

In the previous embodiment, in sub step (b.ii), the culturing agents comprises IL-2, IL-15, and IL-21 and JQ1.

In a preferred embodiment, in sub step (b.ii), the cell culture is cultured for 2-9 days.

In embodiments of the method of the present invention, in step (a), the culturing agents comprises a bispecific antibody, anti-PD-1, anti-TIM-3, anti-CTLA-4, anti-TIGIT, IDO1 inhibitor, adenosine 2A receptor antagonist, IL-2, IL-15, and IL-21. In a preferred embodiment, the bispecific antibody is a CD3/CD123 bispecific antibody.

In embodiments, the method of the present invention further comprises, after step (c), the following step:
(d) washing the cell culture obtained in step (d) for removing agents remaining in the cell culture.

In embodiments of the method of the present invention, the immune cells and the live cancer cells are from the same subject, but from different tissues.

In embodiments of the method of the present invention, the immune cells are derived from bone marrow from a subject without bone marrow tumor infiltration.

In embodiments of the method of the present invention, the immune cells are derived from a first subject and the live cancer cells are derived from a second subject.

In embodiments of the method of the present invention, the first subject and the second subject suffer the same type of cancer.

In embodiments of the method of the present invention, the immune cells are derived from a first subject and the live cancer cells are derived from cancer cell lines.

In embodiments, the method of the present invention further comprises, after step (d) or (e), quantifying traces of cancer cells.

According to this embodiment, traces of cancer cells are quantified in the cell therapy product to ensure absence of live cancer cells in said T cell therapy product.

In a preferred embodiment of the method of the present invention, quantification of traces of cancer cells is carried out by a flow cytometry-based minimal residual disease (MRD) quality test; and qualifying the immune cells as suitable for immunotherapy if no cancer cells have been detected above a detection limit of 0.01% in the MRD quality test.

CAR-T manufacturing methods in less than a week, avoiding the chemotherapy cycle, are currently under development. However, in said approaches, it has not been possible to administer the CAR-T cells to the patient in less than a month, in spite of using manufacturing methods of less than 1 week. This is due to the complex logistics associated to centralized manufacturing, requiring first the patient sample to be cryopreserved to be shipped to a centralized manufacturing facility, where it has to wait for a manufacturing slot, and after the CAR-T is produced it is cryopreserved to be shipped to the hospital for patient infusion.

Manufacturing T cell therapies faces a big challenge to reach all patients in need. Complex logistical steps need to be simplified, and standardized, before automation can be used to scale up production capacity.

In embodiments, the method of the present invention is carried out in an automated cell manufacturing system.

According to this embodiment, cell therapies are manufactured in the hospital by automated cell manufacturing systems with simplified and standardized steps. The method of the present invention can be easily implemented at the point-of-care hospital using automated cell manufacturing systems within only 7 days. Examples of automated cell manufacturing systems include, but are not limited to, CliniMacs Prodigy, Cytiva Xuri W25 rocking platform, and Wilson-Wolf G-Rex gas-permeable bioreactor.

The T cells, such as TILs, CAR-T, TCR-T, obtained according to the method of the present invention, expanded in the presence of live tumor cells, are already activated and have been avidly killing cancer cells during expansion and thus upon infusion in the patient, while CAR T and TILs grown in isolation present a time-lag.

In embodiments of the method of the present invention, the sample is from a subject having a cancer selected from the group consisting of: B cell acute lymphoblastic leukemia (ALL), hairy cell leukemia, non-Hodgkin's lymphoma, Hodgkin's lymphoma, B cell lymphoma, mantle cell lymphoma (MCL), diffuse large B cell lymphoma (DLBCL), follicular lymphoma, marginal zone B cell lymphoma, acute myeloid leukemia (AML), chronic lymphocytic leukemia (CLL), chronic myelogenous leukemia (CML), chordoma, chronic myeloproliferative neoplasm, craniopharyngioma, embryonal tumor, medulloblastoma, Burkitt's lymphoma, lymphoplasmacytic lymphoma, myelodysplastic syndrome (MDS), multiple myeloma, adrenocortical carcinoma, astrocytoma, gastrointestinal neuroendocrine tumor, atypical teratoid/rhabdoid tumor, breast cancer, colorectal cancer, ovarian cancer, ovarian germ cell tumor, rectal cancer, stomach cancer, testicular cancer, anal region cancer, uterine cancer, endometrial cancer, colon cancer, renal-cell carcinoma, liver cancer, gallbladder cancer, intrahepatic bile duct cancer, heart tumor, Langerhans cell histiocytosis, laryngeal cancer, pharyngeal cancer, mesothelioma, midline tract carcinoma, non-small cell lung cancer, small cell lung cancer, lung cancer, pleuropulmonary blastoma, pulmonary inflammatory myofibroblastic tumor, tracheobronchial tumor, small intestine cancer, esophagus cancer, melanoma, intraocular melanoma, retinoblastoma, Kaposi's sarcoma, AIDS-related lymphoma, multiple endocrine neoplasia syndrome, plasma cell neoplasm, myelodysplastic syndrome, myelodysplastic/myeloproliferative neoplasm, neuroblastoma, paraganglioma, endocrine system cancer, thyroid gland cancer, parathyroid gland cancer, thymoma, thymic carcinoma, adrenal gland cancer, bone cancer, pancreatic cancer, pancreatic neuroendocrine tumor, islet cell tumor, skin cancer, head cancer, neck cancer, brain stem glioma, brain tumor, bronchial tumor, central nervous system embryonal tumor, germ cell tumor, childhood central nervous system germ cell tumor, primary central nervous system lymphoma, cervical cancer, extracranial germ cell tumor, extragonadal germ cell tumor, gestational trophoblastic disease, pheochromocytoma, peritoneal cancer, pituitary adenoma, epidermoid cancer, cervix squamous cell cancer, fallopian tubes carcinoma, endometrium carcinoma, ependymoma, vagina carcinoma, soft tissue sarcoma, Ewing sarcoma, osteosarcoma, malignant fibrous histiocytoma, undifferentiated pleomorphic sarcoma of bone, rhabdomyosarcoma, uterine sarcoma, urethra cancer, vulva carcinoma, penis cancer, bladder cancer, kidney cancer, prostate cancer, ureter cancer, renal pelvis carcinoma, vulvar cancer, spinal axis tumor, and central nervous system (CNS) neoplasm.

The present invention also provides immune cells obtainable according to the method of the present invention.

The present invention also provides immune cells of the present invention for use in an immunotherapy for the treatment of cancer.

The immune cells of the present invention can be used for allogenic immunotherapy. There is a large crossover of antigens used by samples from different patients of the same cancer type. Thus, activated immune cells from one patient sample can kill cancer cells from another patient sample of the same cancer type.

In a preferred embodiment, the immunotherapy is an autologous immunotherapy or an allogenic immunotherapy.

In other preferred embodiment of the immune cells for use, the cancer to be treated is selected from the group consisting of: B cell acute lymphoblastic leukemia (ALL), hairy cell leukemia, non-Hodgkin's lymphoma, Hodgkin's lymphoma, B cell lymphoma, mantle cell lymphoma (MCL), diffuse large B cell lymphoma (DLBCL), follicular lymphoma, marginal zone B cell lymphoma, acute myeloid leukemia (AML), chronic lymphocytic leukemia (CLL), chronic myelogenous leukemia (CML), chordoma, chronic myeloproliferative neoplasm, craniopharyngioma, embryonal tumor, medulloblastoma, Burkitt's lymphoma, lymphoplasmacytic lymphoma, myelodysplastic syndrome (MDS), multiple myeloma, adrenocortical carcinoma, astrocytoma, gastrointestinal neuroendocrine tumor, atypical teratoid/rhabdoid tumor, breast cancer, colorectal cancer, ovarian cancer, ovarian germ cell tumor, rectal cancer, stomach cancer, testicular cancer, anal region cancer, uterine cancer, endometrial cancer, colon cancer, renal-cell carcinoma, liver cancer, gallbladder cancer, intrahepatic bile duct cancer, heart tumor, Langerhans cell histiocytosis, laryngeal cancer, pharyngeal cancer, mesothelioma, midline tract carcinoma, non-small cell lung cancer, small cell lung cancer, lung cancer, pleuropulmonary blastoma, pulmonary inflammatory myofibroblastic tumor, tracheobronchial tumor, small intestine cancer, esophagus cancer, melanoma, intraocular melanoma, retinoblastoma, Kaposi's sarcoma, AIDS-related lymphoma, multiple endocrine neoplasia syndrome, plasma cell neoplasm, myelodysplastic syndrome, myelodysplastic/myeloproliferative neoplasm, neuroblastoma, paraganglioma, endocrine system cancer, thyroid gland cancer, parathyroid gland cancer, thymoma, thymic carcinoma, adrenal gland cancer, bone cancer, pancreatic cancer, pancreatic neuroendocrine tumor, islet cell tumor, skin cancer, head cancer, neck cancer, brain stem glioma, brain tumor, bronchial tumor, central nervous system embryonal tumor, germ cell tumor, childhood central nervous system germ cell tumor, primary central nervous system lymphoma, cervical cancer, extracranial germ cell tumor, extragonadal germ cell tumor, gestational trophoblastic disease, pheochromocytoma, peritoneal cancer, pituitary adenoma, epidermoid cancer, cervix squamous cell cancer, fallopian tubes carcinoma, endometrium carcinoma, ependymoma, vagina carcinoma, soft tissue sarcoma, Ewing sarcoma, osteosarcoma, malignant fibrous histiocytoma, undifferentiated pleomorphic sarcoma of bone, rhabdomyosarcoma, uterine sarcoma, urethra cancer, vulva carcinoma, penis cancer, bladder cancer, kidney cancer, prostate cancer, ureter cancer, renal pelvis carcinoma, vulvar cancer, spinal axis tumor, and central nervous system (CNS) neoplasm.

In a preferred embodiment of the immune cells for use of the present invention, the cancer to be treated is a hematological malignancy. In a more preferred embodiment, the cancer to be treated is acute myeloid leukemia (AML).

As used in this specification and the appended claims, the singular forms "a", "an", and "the" include plural referents unless the content clearly dictates otherwise.

The term "comprise", "comprising" and their variants, throughout the description and the claims, includes, specifically, the term "consisting" or "consisting of".

### BRIEF DESCRIPTION OF DRAWINGS

Fig. 1. TIL expansion with live tumor cells.
Fig. 2. Adding PD-1 and IL-2+IL-15+IL-21 to 3 AML samples activated with a CD3xCD123 (CB) bispecific antibody enhances cancer killing substantially in 2/3 samples.
Fig. 3. TIL activation and expansion. To wells with the AML sample, medium plus a cocktail composed of: CD3xCD123 bispecific antibody (100 ng/ml) + adenosine 2A receptor antagonist (1 µM) + IDO1 inhibitor (2.5 µg/ml) + IL-2 (50 IU/ml) + IL15 (10 ng/ml) + IL21 (20 ng/ml) + PD-1 (10 µg/ml) + TIM-3 (2.5 µg/ml) + TIGIT (2.5 µg/ml) + CTLA-4 (2.5ug/ml) was added (represented as "1"). At day 5, medium was changed and a new cocktail was added composed of IL-2 (50 IU/ml) + IL-15 (10 ng/ml) + IL-21 (20 ng/ml) + JQ1 (0,0313µM).
Fig. 4. Double isolation with magnetic beads removing AML cells following by selection T cells efficiently removes any AML cells left alive as judged by MRD Quality Control criterion. Left panel, an example for one sample.
Fig. 5. TIL expansion at 120h at different IL-2 doses using a cocktail composed of CD3xCD123 bispecific antibody (100 ng/ml) + IL-15 (10 ng/ml) + IL-21 (20 ng/ml) + PD-1 (10 µg/ml).
Fig. 6. T cell memory subtype distribution in the TILs manufactured by the method of the present invention.
Fig. 7. IFN-γ and TNF-α secreted by the produced TILs. Left panel shows lovance Biotherapeutics TILs, middle panel shows clonal neoantigen TILs of Achilles Therapeutics and right panel shows TILs manufactured by the method of the present invention.
Fig. 8. CD107a expression in TILs manufactured for 7 days by the method of the present invention.
Fig. 9. Low % activated T cell exhaustion measured by intracellular expression of the TOX protein. The TOX+ TILs from a representative AML at times zero, 120h (5 days) and 168h (7 days) are represented.
Fig. 10. Confirmation that there is no detectable CD3xCD123 bispecific antibody left in washed TILs activated by the bispecific antibody that kill tumor cells. Top panel shows binding of a biotin-linked antibody against the CD3xCD123 bispecific antibody. Labeling of CD3xCD123 incubated AML cells is detected in the presence of streptavidin vs its absence. Middle panel shows that after washing the bispecific antibody is undetectable. Lower panel shows that these TILs without detectable CD3xCD123 bispecific antibody can kill autologous AML cells.
Fig. 11. Activity of activated TILs from an AML bone marrow patient sample killing AML cells in 24 h incubation from 3 patients was assayed in three cases: 1) autologous AML cells, 2) AML bone marrow cells stably expanded in a PDX mouse model, and 3) the immortalized cell line MOLM13 derived from an AML patient bone marrow sample.
Fig. 12: Activated and expanded TILs from an ovarian cancer sample effectively killed tumor cells from the immortalized pancreatic tumor cell line BXPC3. Point 1 represents only BXPC3, point 2 represents BXPC3 + PD-1, curve 3 represents BXPC3 + TILs, curve 4 represents BXPC3 + TILs + PD-1.
Fig. 13. Ex vivo testing TIL vs CART vs CAR-TIL on AML PDX tumor cells.
Fig. 14. Bone marrow distribution of TILs or CARTs at days 2 & 4 in PDX models.
Fig. 15. Spleen distribution of TILs or CARTs at days 2 & 4 in PDX models.
Fig. 16. Peripheral blood distribution of TILs or CARTs at days 2 & 4 in PDX models.

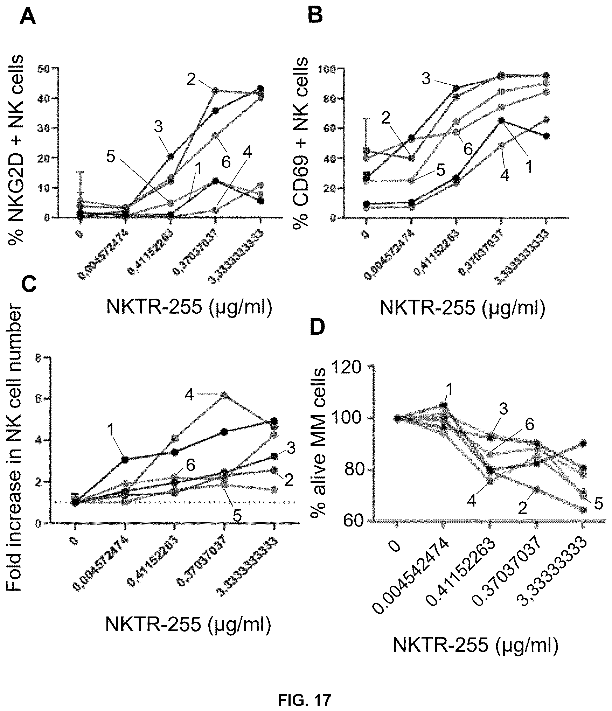

Fig. 17. NKTR-255 induces activation and growth of bone marrow NK cells, reducing survival of autologous MM cells. Whole bone marrow samples of 6 NDMM patients incubated for 120 hours with 4 doses of NKTR-255 induced a dose-dependent activation profile of NK cells (A, B), expansion of NK cell populations (C), and a reduction of MM cell viability (D) as compared to control in an autologous setting using an automated flow cytometry platform for these assessments. Points marked as "1" correspond to patient 1 and E:T ratio 0.13; points marked as "2" correspond to patient 2 and E:T ratio 0.09; points marked as "3" correspond to patient 3 and E:T ratio 0.125; points marked as "4" correspond to patient 4 and E:T ratio 0.362; points marked as "5" correspond to patient 5 and E:T ratio 0.207 and points marked as "6" correspond to patient 6 and E:T ratio 0.053.

### DESCRIPTION OF EMBODIMENTS

### Example 1. Incubation of TILs with live tumor cells and a cocktail of culturing agents

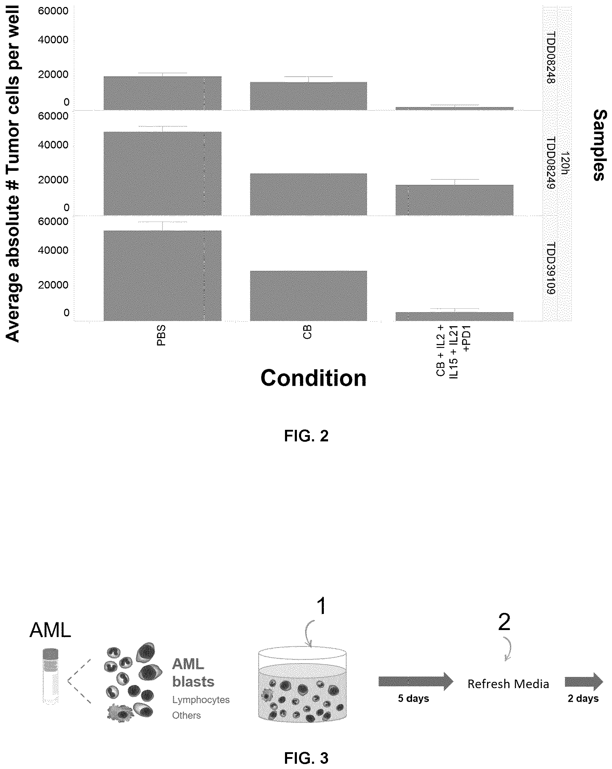

This example shows the significant improvement in *ex vivo* cancer-killing incubation of T cells by adding immune checkpoint inhibitors, immune modulatory agents, and cytokines. The absolute number of tumor cells per well for 3 AML samples incubated for 120 h with different agents were measured: 1) PBS media as control (1st column), 2) adding the bispecific antibody CD3xCD123 (100 ng/ml) from Creative Biolabs (CB) that activates the T cells inducing tumor cell killing (2nd column), and 3) CB plus IL-2 (50 IU/ml) + IL-15 (10 ng/ml) + IL-21 (20 ng/ml) + PD-1 (10 µg/ml) (column 3). Adding the PD-1 immune checkpoint inhibitor and the cytokines IL-2+IL-15+IL-21 (column 3) enhances cancer cell killing substantially for 2/3 samples (top and bottom samples). The results are shown in **Fig. 2**.

### Example 2. No residual tumor cells in final product

To validate that there are no residual tumor cells left alive in the final TIL product to be administered to patients, 8 AML samples were selected, and the manufacturing method of the invention was applied. The scheme of the activation and expansion method is shown in **Fig. 3**. The activation and expansion method is detailed below.

### Day 1

8 cryopreserved AML samples were thawed, a cell count performed by flow cytometry to measure the viability and basal E:T ratios. Sample was incubated in 96-well plates at a minimum of 500 T cells per well, with their corresponding number of tumor cells according to each sample E:T ratio. For each plate, 5 wells as controls were filled adding only media, 60 wells with AML sample. To the 60 wells with AML sample, medium plus a cocktail was added. The cocktail was composed of: CD3xCD123 bispecific antibody (100 ng/ml) + adenosine 2A receptor antagonist (1 µM) + IDO1 inhibitor (2.5 µg/ml) + IL-2 (50 IU/ml) + IL-15 (10 ng/ml) + IL-21 (20 ng/ml) + PD-1 (10 µg/ml) + TIM-3 (2.5 µg/ml) + TIGIT (2.5 µg/ml) + CTLA-4 (2.5 µg/ml).

The commercial reagents used were the following: X-Vivo15 (catalog number H3BE02-061Q, Media Cultek); adenosine 2A receptor antagonist 2-(Furan-2-yl)-7-phenethyl-7H-pyrazolo[4,3-e][1,2,4]triazolo[1,5-c]pyrimidin-5-amine (commercial name SCH58261, CAS No.: 160098-96-4, catalog number S8104, Selleck); human IL-2 IS premium grade (catalog number 130-097-746, Miltenyi Biotec); human IL-15, premium grade (catalog number 130-095-764, Miltenyi Biotec); LAG-3 Antibody (L4-PL33) BSA free (catalog number NBP1-97657, Novus); ultra-Leaf Purified anti-human CD366 (TIM-3) Antibody Clone F38-2E2 (catalog number 345010, BioLegend); ultra-LEAF Purified anti-human CD152 (CTLA-4) Antibody Clone L3D10 (catalog number 349932, BioLegend); ultra-LEAF Purified anti-human TIGIT Antibody Clone A15153A (catalog number 613704, BioLegend); purified anti-human CD159a (NKG2A) Antibody Clone S19004C (catalog number 375102, BioLegend); recombinant human IL-21 (catalog number 200-21, Peprotech); CD3xCD123 bispecific antibody (Creative Biolabs); IDO1 inhibitor (1-methyl-D-tryptophan, commercial name indoximod; catalog number S7756, CAS NO. 110117-83-4).

### Day 5

3 AML + cocktail wells and 3 control wells were evaluated for viability and activity. Medium and cocktail in AML wells was changed. The new cocktail was composed of IL-2 (50 IU/ml) + IL-15 (10 ng/ml) + IL-21 (20 ng/ml) + JQ1 (0,0313 µM).

The reagent JQ1 is the compound:
(S)-(+)-tert-Butyl 2-(4-(4-chlorophenyl)-2,3,9-trimethyl-6H-thieno(3,2-f)(1,2,4)triazolo(4,3-a)(1,4)diazepin-6-yl)acetate (CAS No.: 1268524-70-4).

JQ1 is an inhibitor of bromodomain and extra-terminal motif (BET) proteins and has been shown to extend CAR T live *in vivo.*

### Day 7

3 AML + cocktail wells, and 2 control wells were evaluated to measure activity in terms of viability of T and tumor cells and expansion of TIL cells. All AML + cocktail wells were pooled together. T cell isolation that efficiently removes all detectable AML cells were carried out. The whole sample contained autologous AML cells; isolated T cells may include trace amounts of AML cells that should be removed. To ensure no AML cells are left in final product a double isolation with magnetic beads and a MRD quality control test were carried out.

Double isolation with magnetic beads: First isolation to remove AML cells with magnetic beads bearing AML selective target CD33, and second isolation selecting T cells with CD4+CD8+ magnetic beads.

MRD as Quality Control test to confirm there are no traces of AML cells in the final product. **Fig. 4** shows a patient sample with 70% starting AML cells that after our manufacturing method and double isolation, no detectable levels of AML were found (10⁻⁵ in **Fig. 4**, left panel). This MRD detection was repeated on 8 AML samples, and all showed undetectable levels of AML cell ranging from 10⁻⁴ to 10⁻⁶ (**Table 1**).

**Table 1**

| Sample | Basal % tumor | % tumor after 7 days production | % T cells after 7 days production | 2-isolation MRD |
|---|---|---|---|---|
| 1 | 88 | 0 | 100 | 10⁻⁴ |
| 2 | 70 | 15 | 85 | 10⁻⁵ |
| 3 | 73 | 0 | 100 | 10⁻⁵ |
| 4 | 69 | 0 | 100 | 10⁻⁶ |
| 5 | 95 | 0 | 100 | 10⁻⁶ |
| 6 | 12 | 0 | 99.5 | 10⁻⁴ |
| 7 | 80 | 0 | 99.5 | 10⁻⁵ |
| 8 | 26 | 0 | 99.8 | 10⁻⁵ |

This example shows that no traces of AML cells could be detected in 8 AML samples in the manufactured final product using the standard MRD assays as Quality Control test with its clinically approved standard thresholds. For 7/8 samples all tumor cells were killed by day 7, except one at 15% resistant AML cells. This subset of resistant AML cells was efficiently removed.

### Example 3. TIL expansion at different IL-2 doses

Since IL-2-expanded TILs lose their TSA T cells, lower doses of IL-2 vs the standard dose of 500 IU/ml were evaluated. TIL expansion at 120h at different IL-2 doses using a cocktail (CD3xCD123 bispecific antibody (100 ng/ml) + IL-15 (10 ng/ml) + IL-21 (20 ng/ml) + PD-1 (10 µg/ml)) was determined. The results are shown in **Fig. 5**. The fold increase does not change substantially at the different doses. The low dose of 50 IU/ml for IL-2 was selected, 10-times lower than the standard dose, achieving a 30-fold expansion of the total number of activated TIL cells.

On the basis of a cocktail composed of CD3xCD123 bispecific antibody (100 ng/ml) + IL-2 (50 IU/ml) + IL-15 (10 ng/ml) + IL-21 (20 ng/ml) + PD-1 (10 µg/ml), the results in 96 well plates were extrapolated to clinical manufacturing. Using the expected volume for clinical manufacturing of a 50 ml bone marrow sample, 1.2 billion activated TILs on average can be generated in 7 days. This is at least 2 doses of clinically relevant amounts of TILs based on KYMRIAH CAR T dosage (0.6-6×10⁶). Current trends favor lower CAR T doses of 100 M or less, and thus the manufacturing capacity of the method of the present invention exceeds therapeutic requirements and lower ml of bone marrow could be used.

### Example 4. T cell memory subtype distribution in the produced TILs

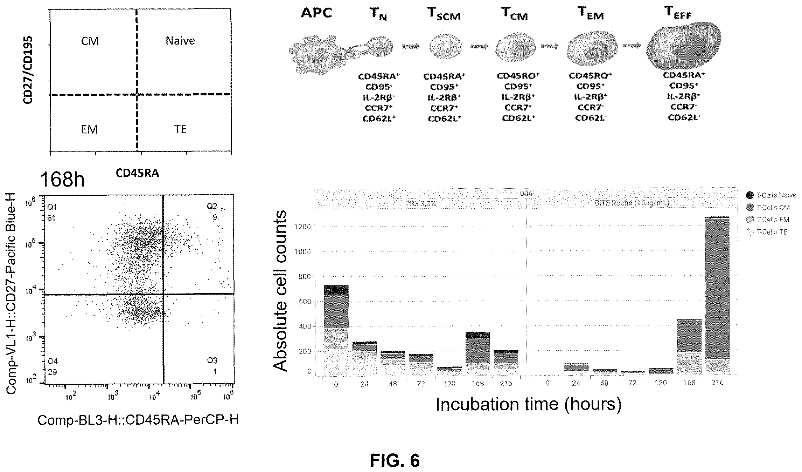

The manufactured activated TIL product has been characterized to ascertain whether it is suitable for cell therapy. The T cell memory subtype distribution in the produced TILs for AML samples were measured. The results are shown in **Fig. 6**. The evolution of memory subtypes is shown with the flow cytometry markers used (top right panel of **Fig. 6**). The quadrant distribution of memory types as they evolve from naive to terminal effectors is shown in the top left panel of **Fig. 6**: top right Naive, top left Central Memory (CM), bottom left Effector Memory (EM), bottom right Terminal Effectors (TE). Lower left panel of **Fig. 6** shows TILs from a representative AML sample where CM and EM are populated, few % of naïve T cell remain, and very few TE can be appreciated. The distribution of these memory subtypes over incubation time in terms of the absolute counts per well are shown in bottom right panel of **Fig. 6**: left graph shows the control incubation without CD3xCD123 bispecific antibody, and right graph the values incubating with the CD3xCD123 bispecific antibody. At 120h with the bispecific antibody a significant T cell expansion as an increase in absolute counts is observed. The expansion contains mostly T cells with CE and EM phenotypes, which are the desired phenotypes for cell therapy, and a very low % of TEs, actually lower than control TILs. Terminal Effectors represent T cells that have a potent cytotoxic potential but have lost the ability to proliferate. In terms of T cell therapy, it means TEs can kill tumor cells in the patient body but this killing is temporal because they cannot proliferate any longer, and thus cannot provide to the patient the long term remissions sought by these T cell therapies. This example shows that the activated TILs manufactured by the method of the present invention have the desired memory phenotype for a T cell therapy.

### Example 5. IFN-γ and TNF-α secreted by the produced TILs

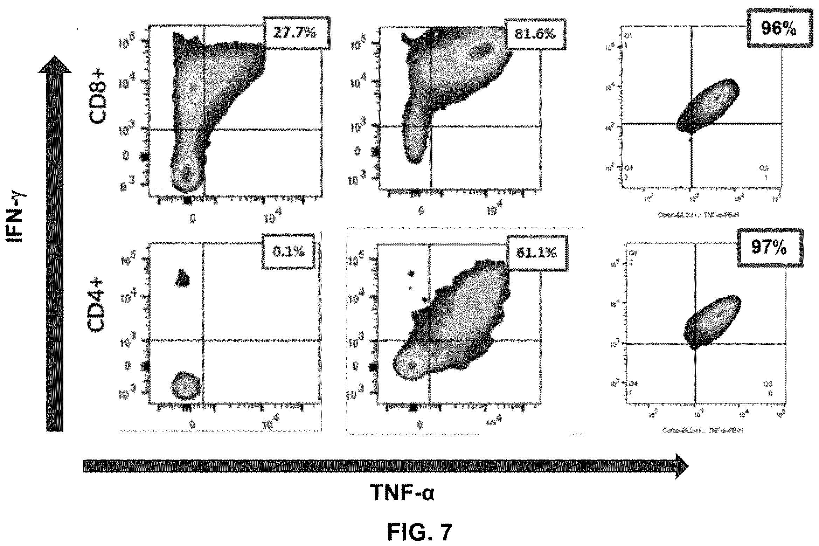

To evaluate the % of activated TILs able to kill cancer cells, the % of CD8+ and CD4+ TILs that secrete both IFN-γ and TNF-α have been analyzed. The TILs produced by the method of the present invention have been compared with TILs from 2 TIL therapy companies, lovance Biotherapeutics and Achilles Therapeutics. The results are shown in **Fig. 7**. Left panel of **Fig. 7** shows TILs of lovance Biotherapeutics, composed only of CD8+ which show a 27.7% IFN-γ/TNF-α + TILs. Middle panel of **Fig. 7** shows TILs of Achilles Therapeutics, with 81.6% for CD8+ and 61.1% for CD4+. Because a typical CD8:CD4 distribution is around 75% CD4+, the overall % would be 71%. Right panel of **Fig. 7** shows TILs manufactured by the method of the present invention, incubating with the live tumor cells, showing the highest value with 96% for CD8+ and 97% for CD4+, because CD4+ represent typically 75% of activated TILs the overall value would be 96.5%. Thus, higher % of tumor killing activated TILs for T cell therapy are obtained by the method of the present invention with respect to activated TILs of the prior art.

### Example 6. CD107a expression by the produced TILs

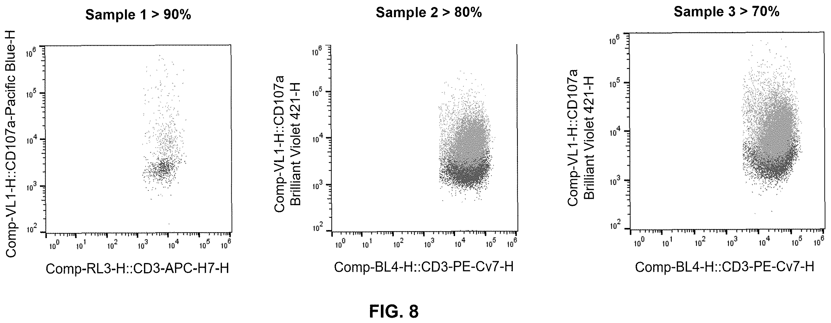

To evaluate the % of cancer-killing T cells in the cell therapy product after 7 days manufacturing, the % of CD107a positive TILs was measured. The marker CD107a on the cell surface of CD8+ and CD4+ T cells following activation indicate degranulation, which implies a killing activity having secreted cytotoxic cytokines, and is normally concordant with production of intracellular IFN-γ. The CD107a marker expression in activated TILs in 3 representative AML samples has been determined. The results are shown in **Fig. 8**. CD107a expression shows a similar pattern as shown in **Fig. 7**. X-axis of **Fig. 8** represent T cells identified by a CD3 marker, and y-axis of **Fig. 8** represents T cells identified by CD107a.

For sample 1, the counts for activated T cells stained with CD107a is 438 and unstained activated T cells is 601. For sample 2, the counts for activated T cells stained with CD107a is 19904 and unstained activated T cells is 22214. For sample 3, the counts for activated T cells stained with CD107a is 13101 and unstained activated T cells is 16865.

The TILs not stained with CD107a are the control, and TILs stained with CD107a that show significantly higher CD107 staining vs control represent the CD107a positive TILs. Quantitative analyses show CD107a positive TILs values of > 90% for sample 1 (left), > 80% for sample 2 (middle), and > 70% for sample 3. These high values are considered good values for a T cell therapy.

### Example 7. Expression of the TOX protein by the produced TILs

One of the concerns for a T cell therapy product expanded *in vitro* is the exhaustion of the activated T cells, which signals they these cells are losing their cytotoxic abilities, normally as a result of chronic (excessive) stimulation. T cell exhaustion can be measured by expression of immune check point inhibitors, but our TILs show high levels of IFN-γ secretion (**Fig. 7**) and this is known to increase expression of immune check point inhibitors without meaning that T cells are exhausted because they can kill efficiently. Thus, intracellular expression of the TOX protein was measured by intracellular antibody staining, that is a reliable method to evaluate % of exhausted T cells.

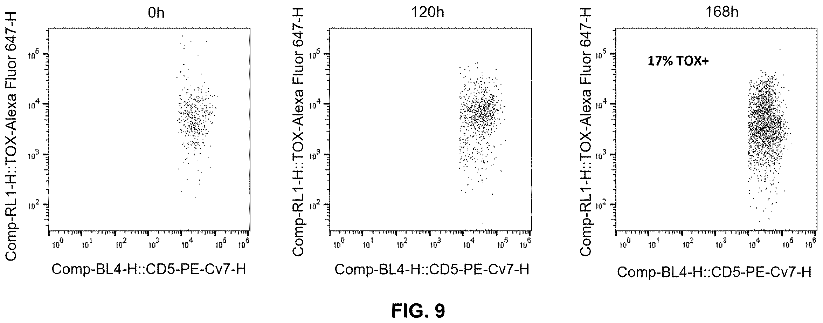

The TOX+ TILs from AML samples at times zero, 120h (5 days) and 168h (7 days) have been measured. The results are shown in **Fig. 9** for a representative sample. The final T cell therapy product at 7 days show a small 17% of TOX+ TILs presumably exhausted. This % is reasonable for a T cell therapy product in that the large majority (83%) of activated TILs are not exhausted.

### Example 8. Removal of remaining agents in the cell culture

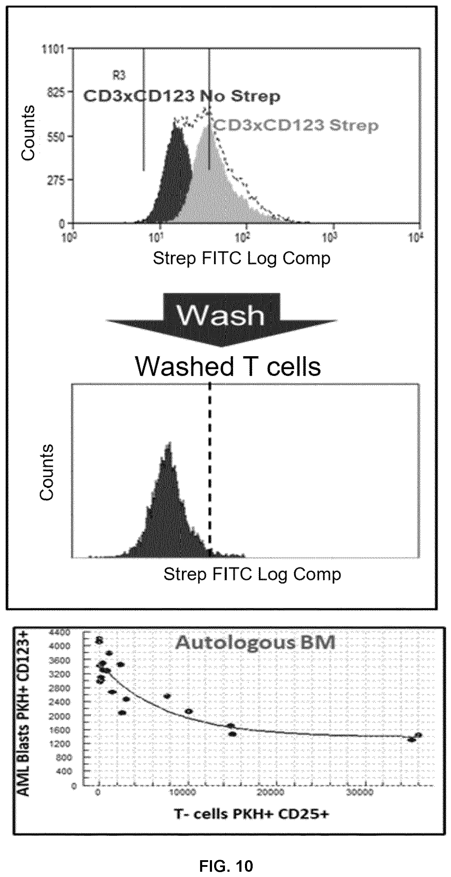

In this example, a wash step was carried out before administration of TILs to the patient, for removing all reagents from the cocktail, since these reagents include mouse or rabbit antibodies that may cause an antigenic reaction. **Fig. 10** shows that the wash step removes the bispecific antibody CD3xCD123 for an AML sample post incubation. Top panel of **Fig. 10** shows binding of a biotin-linked antibody against the CD3xCD123 bispecific antibody. Labeling of CD3xCD123 incubated AML cells is detected in the presence of streptavidin vs its absence. Middle panel of **Fig. 10** shows that after washing the bispecific antibody is undetectable. Lower panel of **Fig. 10** shows that these TILs without detectable CD3xCD123 bispecific antibody can kill autologous AML cells.

### Example 9. Allogenic TIL immunotherapy

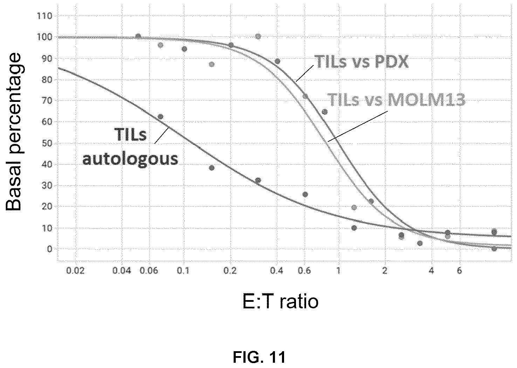

The TILs of the present invention can be used for allogenic immunotherapy. Activity of activated TILs from an AML bone marrow patient sample killing AML cells in 24 h incubation from 3 patients was assayed in three cases: 1) autologous AML cells, 2) AML bone marrow cells stably expanded in a PDX mouse model, and 3) the immortalized cell line MOLM13 derived from an AML patient bone marrow sample. The results are shown in **Fig. 11**. **Fig. 11** shows that although autologous killing is more potent than killing the two allogenic samples (PDX and MOLM13), the activated TILs can kill all AML cells in both autologous and allogenic AML cells. Thus, the TILs manufactured by the method of the present invention can be used in allogenic TIL immunotherapies. **Fig. 11** also demonstrates that it is possible to incubate TILs with immortalized cell lines when there are insufficient patient tumor cells available.

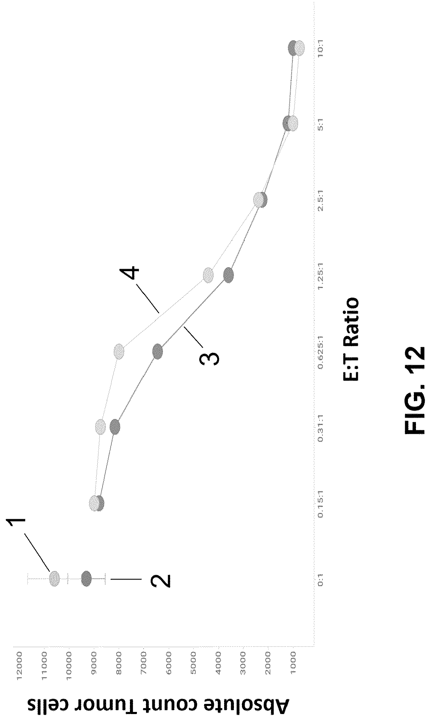

In an additional allogenic killing assay, TILs from an ovarian cancer patient sample activated and expanded effectively killed tumor cells from the immortalized pancreatic tumor cell line BXPC3. The results are shown in **Fig. 12**. **Fig. 12** also shows the dose response curve in the presence of the PD-1 antibody.

### Example 10. Enhanced accumulation in tumor tissues of TILs vs CAR-PB T cells days 2-4

To test whether manufactured TILs with live tumor cells claimed herein have an enhanced migration/accumulation to the tumor tissues, it is selected a PDX mouse model of AML that is stable over generations showing significant infiltration in bone marrow, the physiological tissue for AML cells in patients. The PDX AML sample had the following markers by flow cytometry: Flow Cytometry 81% Blasts with phenotype CD34-/+d (23%) CD117+ CD45+d MPO+ HLA-DR+het (más débil en la población CD34-) CD13+ CD33+ CD11b- CD15- NG2-CD16- CD14- IREM- CD64- CD71+ CD36- CD105- CD19- CD79b- sCD3- cyCD3- CD7+ CD9+ (66%) CD41- CD42b- CD25+ CD123+ CD4+d/- TdT-/+d. This includes positive for CD123, which was confirmed in the AML PDX sample for this study, and TILs will be activated with a CD3xCD123 bispecific antibody as in the previous examples.

A CAR T CD123 was generated transducing T cells with a lentivirus vector containing the CAR CD123 construct and the activator domain 41BB purchased from Creative Biolabs. The CAR transduction protocol is described below.

### Day -1: prepare the plate

Medium: X-VIVO 15 + 5% Human serum AB + antibiotics

α-CD3 + α-CD28 antibodies (overnight at 4°C): α-CD3 (1 µg/ml) + α-CD28 (1 µg/ml) in PBS. Plate 100 µl/well p96.

### Day 0

### Ficoll

- 15ml Ficoll + 35ml (10ml blood + 25ml PBS)
- Centrifuge Ω1800 rpm 20 min (ac 6, dec 3)
- Take the mononuclear cells and wash with PBS
- Centrifuge Ω1400 rpm 5 min, resuspend cells in PBS and count

### CD3+ selection

- LD column (Ref:130-042-901, Miltenyi Biotec) and Pan T cell Isolation Human kit (Ref: 130-096-535, Miltenyi Biotec)
- Add 40 µl of PBE/RINSE per 10 million cells
- Add 10 µl of Pan T cells Biotin-Antibody Cocktail per 10 million cells
- Mix and incubate during 10 min at 4°C
- Add 30 µl of PBE/RINSE per 10 million cells
- Add 20 µl of Pan T Cell MicroBead Cocktail per 10 million cells
- Mix and incubate during 15 min at 4°C
- Wash LD column with 2 ml of PBE/RINSE
- Add cells to the column
- Wash again the column with 3ml of PBE/RINSE and collect the elute cells (unstained CD3+ cells)
- Centrifuge cells Ω 1400rpm 5 min. Wash with PBS and count the cells

### CD3+ culture conditions

- Remove the antibody from the plates
- Wash the plate with PBS (100 µl/well) (3X)
- Incubate each well with 100 µl of PBS-3%FBS (15 min at RT)
- Remove the PBS and plate the cells at 2mill/ml in 200ul. The medium used is X-VIVO + 5% AB serum + antibiotics + IL-2 (500 IU/ml) + IL-15 (10 ng/ml). Instead of IL-2, IL-7 (10ng/ml) can be used

### Day 2-3: CAR transduction

- The day before, add 2 µg/cm² of retronectin to a plate and store at 4°C. Also, the plate can be stored with retronectin during 4h at RT the same day.
- Remove cells of the plate and centrifuge during 5 min 1400 rpm.
- Count the cells and resuspend at 2 millions/ml.
- Transduce T cells with CAR lentivirus at MOI 5. Plate 1 million cells per p24 well.
- Centrifuge the plate during 20 min at 2000 rpm.

### Following day: remove virus

- Remove cells from the plate
- Centrifuge at 1400 rpm 5 min
- Plate cells at 2 millions/ml and expand them changing the medium every 3 days

Healthy donor T cells (CART HD) were used as a control, given the known difficulties in producing CART from AML peripheral blood patients (CART AML).

To produce large numbers of activated TILs from BM using the method of the present invention and sufficient T cells from PB to produce the CART AML the following patient was selected: an AML patient from which large volumes of bone marrow (BM) and peripheral blood (PB) samples were obtained. This sample had cryopreserved 320M of BM and 330M of PB cells both with a small 3.3% of T cells. The CAR CD123 was also transduced on the 7-day produced BM activated TILs to generate the CAR TIL. The characteristics of these T cells are described below:
CART HD: CAR CD123 on healthy donor T cells. 450M CART HD with 77% CAR+ cells were produced, measured with a fluorescently labelled IL3 (the CD123 ligand) purchased from Creative Biolabs. (450M T cells were obtained and 77% of cells were CAR+).
CART AML: CAR CD123 on PB T cells from the AML patient selected with a high volume sample. 330M PB cells were at the start but the majority (99%) of the T cells were lost in the isolation of T cells with magnetic CD3-beads from Milteny. Isolated T cells were activated adding standard CD3/CD28 beads. 19M T cells were obtained. 55 days in culture was necessary to produce this level of T cells. 54,1% of cells were3 CAR+, 100% of cells were CD4+. Phenotype by flow cytometry is the following: 43% TIM3+; 3.5% LAG3+; 42% PD1+; 7.8% NKG2A+; 63.2% Teffector; 20% Tscm; 11.4% Tern; 0% Tern; 44.7% CD25+; 100% CD2+; 68.6% KLRG1+; 77.4% CD28+; 8.1% CD27+; and 4.8% CD57+. High % of immune check points (TIM3, PD1) were obtained, high % of terminal effectors (63%) were also obtained, and senescence marker KLRG1 was 68.6%.
TIL: produced from AML sample following the method of the present invention.
CAR-TIL: Produced transducing the TILs above with the CD123 CAR lentivirus vector described above during 8 days after TIL 7 day production. 29.8 M T cells were obtained. Overall 51 % of T cells were CAR+. However, CD4+ represented 71 % of TILs of which 69% were CAR+, while CD8+ represented 16% of TILs of which only 11% were CAR+.

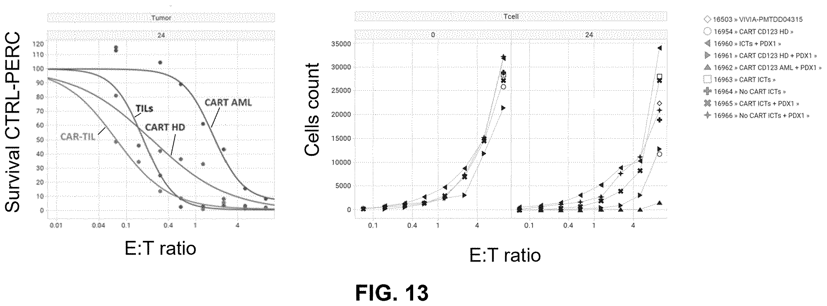

The *ex vivo* activity of these 4 T cell populations against AML cells from the PDX mice model was evaluated. The results are shown in **Fig. 13**.

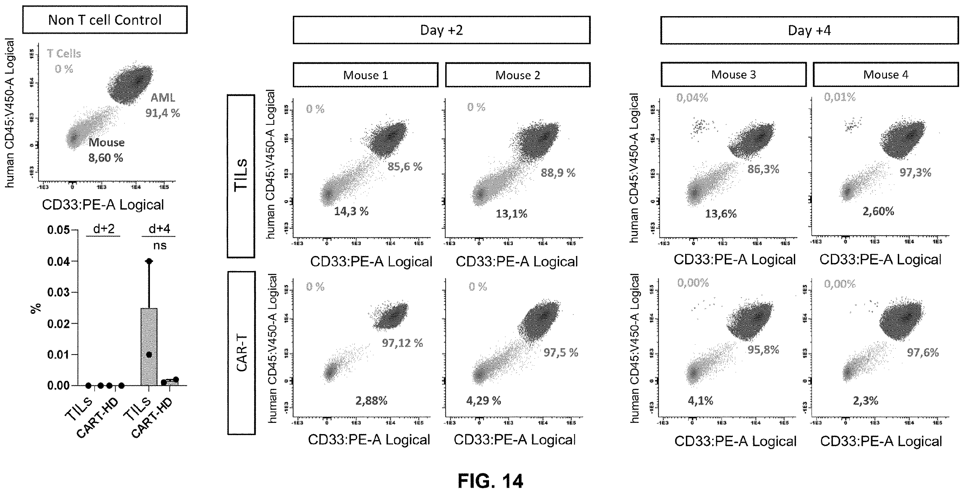

Left panel of **Fig. 14** shows tumor cell killing dose responses at 24h, showing a similar activity observed for CAR-TIL, CART HD, or TILs, while CART AML showed a substantial lower activity. Right panel shows the T cell populations at 0h and 24h. Initially (time 0h) all T cell populations are similar. However, at 24 h the T cells from the CART HD have decreased and the T cells form the CART AML have almost all disappeared, and this lower viability could be masking a higher activity.

These TILs are killing allogenic PDX AML cells in these *ex vivo* experiments. If HLA mismatch was responsible for the tumor cell killing, autologous killing would be lower than allogenic killing. **Fig. 11** showed that these TILs to be administered to the PDX mouse models kill much better the autologous AML cells vs the allogenic PDX or an AML cell line MOLM13. Thus, selective recognition seems to drive AML cell killing by these activated TILs rather than HLA mismatch.

CART HD were used to study the dose of T cells for these PDX mouse models since they were available in large amounts. First, on a mouse model administering an AML cell line MOLM13 with luciferase and GFP (100.000 cells 24h after mouse irradiation), it is administered 2 days later 5, 7.5 and 10 million CART HD cells. Day 21 after MOLM13 administration all 3 control animals have to be sacrificed or died, while all 3 mouse CART HD in all 3 doses were alive showing good CART HD in vivo efficacy. The higher doses 7.5M and 10M induce some weight loss suggestive of graft-vs-host disease, and thus it is decided to use the 5M dose for CART HD. This CART HD was studied in PDX mouse models at 5M/mouse evaluating activity administering the CART HD either 1 week or 2 weeks after PDX administration, and CART HD show good activity in both week +1 and +2 with all 3 mice alive after 28 days when control mice were dead or sacrificed.

Bone marrow infiltration was characterized in the PDX mouse model (N=3) at week +1, +2, +3, +4, by scarifying the mice, extracting bone marrow from all 4 legs, and measuring the % of AML cells relative to control beads by flow cytometry. The results of infiltration by AML cells in bone marrow was approximately 20% infiltration at week +2, 40% at week +3 and 90% at week 4. Week +2 with approximately 20% bone marrow infiltration was selected to study TIL and CART activity. Week +4 with 90% bone marrow infiltration was selected to study TIL vs CART migration, because this mimics the human patient common scenario, and a higher % of infiltration will provide a higher chemokine gradient to attract TILs to the bone marrow.

TILs manufactured by the method of the present invention and CART HD were used to study migration to the tumor site in PDX at week +4. Migration studies were designed by evaluating % TIL or CART cells in tumor sites at 2 days and 4 days. Tumor sites were bone marrow (the physiologically relevant tissue), spleen (where most tumor cells accumulate in this PDX), and peripheral blood. **Fig. 14**, **Fig. 15** and **Fig. 16** show the results for bone marrow, spleen and blood, respectively. Only 2 mice were studied per condition.

Top left of **Fig. 14** shows the control mouse without human T cells. Middle panels of **Fig. 14** show the flow cytometry results for day 2 and right panels for day 4. At day 2 TILs or CARTs are not observed, but at day 4 few TILs were observed and no CARTs. Bottom left panel of **Fig. 14** shows the statistical analysis, at day 4 there are 25 times more ICTs than CARTs in bone marrow, and although this difference is not yet statistically significant it is substantial.

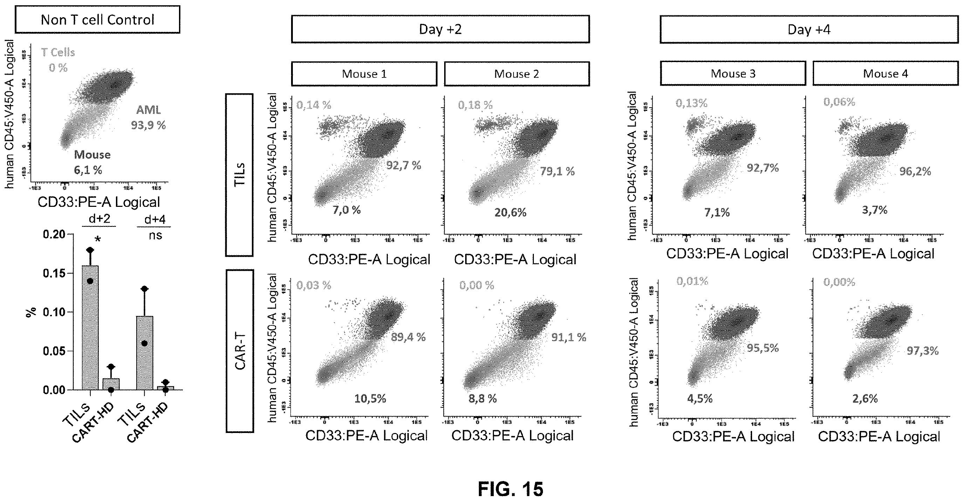

**Fig. 15** shows the same results for spleen, where most of AML tumor cells are concentrated facilitating statistical analysis. Bottom left panel of **Fig. 15** shows the statistical analysis and shows a statistically significant accumulation of ICTs (TILs) over CART at day 2 of 8-fold, and large difference as well at day 4.

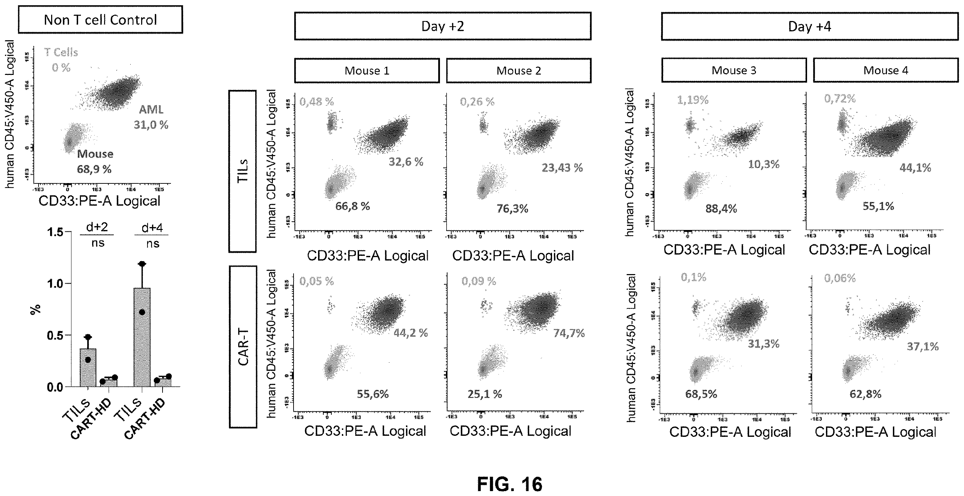

**Fig. 16** shows the results for peripheral blood. Bottom left panel of **Fig. 16** shows the statistical analysis and shows a 10-fold higher % of ICTs vs CARTs, albeit not statistically significant.

In summary, the biodistribution kinetic studies shows that TILs (ICTs) accumulate in tumor tissues faster than CARTs, at a difference of 8-25 fold pending repeating the study in more mice to achieve better statistics. These numbers would mean that at days 2 and 4 the number of TILs vs CART is substantially large at tumor tissues, which may constitute a very important advantage for therapeutic benefit, such as earlier cancer removal of TILs vs CARTs if they have similar *in vivo* activities.

A higher accumulation of TILs at early days has been observed in all tissues, bone marrow, spleen, and blood. This may reflect a higher viability of TILs vs CARTs, since CARTs are known to decrease significantly in circulating numbers the initial days, and if TILs do not disappear similar to CARTs these initial days the result may be an enhanced presence in all tissues.

In this example, a PDX model that was stable over generations and produced reproducible tissue infiltrations was selected. The results show that at day 4 after cell therapy administration there are 25 times more TILs and CAR-TILs vs CAR-PB T cells in bone marrow, spleen, and blood. Because the increased numbers of TILs vs CAR- T cells occurred in all 3 tissues, the results are consistent with either faster migration to tumor sites, and/or higher viability of TILs vs CAR T cells that would explain higher numbers in all tissues with tumor cells.

### Example 11. NK Cell therapy activation and expansion

Multiple myeloma (MM) is characterized by an immunosuppressive microenvironment that enables tumor development. One of the mechanisms of immune evasion used by MM cells is the inhibition of NK cell effector functions; thus, the restoration of NK cell antitumor activity represents a key goal for new immunotherapeutic approaches, increasing tumor cell recognition, avoiding tumor escape, and potentially enhancing the effect of other drugs. Activation and/or expansion of NK cells ex vivo can be facilitated by incubating with live tumor cells, as shown here for a novel agent.

In this example, the potential of NKTR-255, a novel polymer-conjugated human IL-15 to engage the IL-15 pathway and overcome the inhibitory status observed in NK cells from MM patients (Fernandez et al. (2021)) is analyzed. For this purpose, *ex vivo* effects of NKTR-255 on phenotypic features, effector functions and cytotoxicity of NK cells against MM cells have been analyzed.

For ex *vivo* analyses, NK cells from MM patients at different stages of disease were isolated by negative immunomagnetic selection. Cytotoxicity against primary MM cells or cells lines and phenotypic changes after incubation with NKTR-255 for 7 days were assessed through an extensive flow cytometry approach. To assess the effect of NKTR-255 on the NK cell compartment and MM cells in an autologous setting, whole bone marrow (BM) samples from newly diagnosed MM (NDMM) patients were incubated with growing concentrations of NKTR-255 and changes were measured through an automated flow cytometry platform.

Results are shown on **Fig. 17**. Whole bone marrow samples of 6 NDMM patients incubated for 120 hours with 4 doses of NKTR-255 induced a dose-dependent activation profile of NK cells (A, B), expansion of NK cell populations (C), and a reduction of MM cell viability (D) as compared to control in an autologous setting using an automated flow cytometry platform for these assessments.

### CITATION LIST

Besser et al. (2020). Comprehensive single institute experience with melanoma TIL: Long term clinical results, toxicity profile, and prognostic factors of response. Mol Carcinog, 59(7): p. 736-744.

Fernandez RA et al. (2021). P-003: Improving NK cell function in Multiple Myeloma with NKTR-255, a novel polymer-conjugated human IL-15. Clin Lymphoma Myeloma Leuk, 21(2) S40-S41.

Spanjaart et al. (2022). P1462: POPULATION-BASED REAL WORLD RESULTS OF CD19-DIRECTED CAR T-CELL THERAPY FOR PATIENTS WITH RELAPSED OR REFRACTORY LARGE B-CELL LYMPHOMA: A DUTCH CAR T-CELL TUMORBOARD EXPERIENCE. HemaSphere, 6(Suppl): p. 1344-1345.

## Claims

1. An *ex vivo* method for producing immune cells for immunotherapy, wherein the method comprises activating and/or expanding immune cells in a sample comprising said immune cells, the method comprising the following steps:
(a) adding a cell culture medium and culturing agents to the sample, thereby obtaining a cell culture;
(b) culturing the cell culture obtained in step (a), wherein in this step the immune cells are cultured with said culturing agents and with live cancer cells, wherein said live cancer cells were already present in the sample and/or were added in step (a) to the sample; and
(c) enriching the fraction of immune cells in the cell culture obtained in step (b).

2. The method according to claim 1, further comprising, after step (b):
(i) removing cancer cells from the cell culture obtained in step (b).

3. The method according to claim 2, wherein step (i) is carried out by a technique selected from the group consisting of: magnetic beads, FACS sorting, and affinity chromatography.

4. The method according to any one of claims 1 to 3, wherein step (c) is carried out by a technique selected from the group consisting of: magnetic beads, FACS sorting, and affinity chromatography.

5. The method according to any one of claims 1 to 4, wherein the immune cells are selected from the group consisting of: tumor infiltrating lymphocytes (TIL), tumor-specific antigens (TSA) T cells, neoantigen T cells, tumor-associated antigens (TAA) T cells, engineered chimeric antigen receptor T cells (CAR-T), engineered T cell receptor T cells (TCR-T), NK cells, engineered chimeric antigen receptor NK cells (CAR-NK), and engineered T cell receptor NK cells (TCR-NK).

6. The method according to any one of claims 1 to 5, wherein the culturing agents are selected from the group consisting of: immune cell activators, immune checkpoint inhibitors, immune modulatory agents, and cytokines.

7. The method according to any one of claims 1 to 6, wherein the culturing agents are selected from the group consisting of: CD3/CD28 beads, bispecific antibody, anti-PD-1, anti-TIM-3, anti-LAG-3, anti-CTLA-4, anti-TIGIT, anti-NKG2A, IDO1 inhibitor, adenosine 2A receptor antagonist, IL-2, IL-15, IL-21, and JQ1.

8. The method according to any one of claims 1 to 7, wherein, in step (a), the culturing agents comprises a bispecific antibody, anti-PD-1, anti-TIM-3, anti-CTLA-4, anti-TIGIT, IDO1 inhibitor, adenosine 2-antagonist, IL-2, IL-15, and IL-21.

9. The method according to any one of claims 1 to 8, wherein the sample is selected from the group consisting of: tumoral sample, bone marrow, whole bone marrow, blood, whole blood, and peripheral blood.

10. The method according to any one of claims 1 to 9, further comprising, after step (b), genetically engineering the immune cells for obtaining engineered immune cells selected from the group consisting of: engineered chimeric antigen receptor T cells (CAR-T), engineered T cell receptor T cells (TCR-T), engineered chimeric antigen receptor NK cells (CAR-NK), and engineered T cell receptor NK cells (TCR-NK).

11. The method according to any one of claims 1 to 10, wherein step (b) comprises the following sub steps:
(b.i) culturing the cell culture obtained in step (a) for 5 days or less; and
(b.ii) changing the cell culture medium of the cell culture obtained in sub step (i), further adding culturing agents, and culturing the cell culture for at least 2 days.

12. The method according to any one of claims 1 to 10, wherein step (b) comprises the following sub steps:
(b.i) culturing the cell culture obtained in step (a) for 5 days or less;
(b.ii) changing the cell culture medium of the cell culture obtained in sub step (i), further adding culturing agents, and culturing the cell culture for at least 2 days; and
(b.iii) genetically engineering the immune cells for obtaining engineered immune cells selected from the group consisting of: chimeric antigen receptor T cells (CAR-T), engineered T cell receptor T cells (TCR-T), engineered chimeric antigen receptor NK cells (CAR-NK), and engineered T cell receptor NK cells (TCR-NK).

13. The method according to claim 11 or 12, wherein, in sub step (b.ii), the culturing agents comprises IL-2, IL-15, and IL-21 and JQ1.

14. The method according to any one of claims 1 to 13, further comprising, after step (c), quantifying traces of cancer cells.

15. Immune cells obtainable according to the method of any one of claims 1 to 14.

16. The immune cells according to claim 15 for use in an immunotherapy for the treatment of cancer.

17. The immune cells for use according to claim 16, wherein the cancer to be treated is selected from the group consisting of: B cell acute lymphoblastic leukemia (ALL), hairy cell leukemia, non-Hodgkin's lymphoma, Hodgkin's lymphoma, B cell lymphoma, mantle cell lymphoma (MCL), diffuse large B cell lymphoma (DLBCL), follicular lymphoma, marginal zone B cell lymphoma, acute myeloid leukemia (AML), chronic lymphocytic leukemia (CLL), chronic myelogenous leukemia (CML), chordoma, chronic myeloproliferative neoplasm, craniopharyngioma, embryonal tumor, medulloblastoma, Burkitt's lymphoma, lymphoplasmacytic lymphoma, myelodysplastic syndrome (MDS), multiple myeloma, adrenocortical carcinoma, astrocytoma, gastrointestinal neuroendocrine tumor, atypical teratoid/rhabdoid tumor, breast cancer, colorectal cancer, ovarian cancer, ovarian germ cell tumor, rectal cancer, stomach cancer, testicular cancer, anal region cancer, uterine cancer, endometrial cancer, colon cancer, renal-cell carcinoma, liver cancer, gallbladder cancer, intrahepatic bile duct cancer, heart tumor, Langerhans cell histiocytosis, laryngeal cancer, pharyngeal cancer, mesothelioma, midline tract carcinoma, non-small cell lung cancer, small cell lung cancer, lung cancer, pleuropulmonary blastoma, pulmonary inflammatory myofibroblastic tumor, tracheobronchial tumor, small intestine cancer, esophagus cancer, melanoma, intraocular melanoma, retinoblastoma, Kaposi's sarcoma, AIDS-related lymphoma, multiple endocrine neoplasia syndrome, plasma cell neoplasm, myelodysplastic syndrome, myelodysplastic/myeloproliferative neoplasm, neuroblastoma, paraganglioma, endocrine system cancer, thyroid gland cancer, parathyroid gland cancer, thymoma, thymic carcinoma, adrenal gland cancer, bone cancer, pancreatic cancer, pancreatic neuroendocrine tumor, islet cell tumor, skin cancer, head cancer, neck cancer, brain stem glioma, brain tumor, bronchial tumor, central nervous system embryonal tumor, germ cell tumor, childhood central nervous system germ cell tumor, primary central nervous system lymphoma, cervical cancer, extracranial germ cell tumor, extragonadal germ cell tumor, gestational trophoblastic disease, pheochromocytoma, peritoneal cancer, pituitary adenoma, epidermoid cancer, cervix squamous cell cancer, fallopian tubes carcinoma, endometrium carcinoma, ependymoma, vagina carcinoma, soft tissue sarcoma, Ewing sarcoma, osteosarcoma, malignant fibrous histiocytoma, undifferentiated pleomorphic sarcoma of bone, rhabdomyosarcoma, uterine sarcoma, urethra cancer, vulva carcinoma, penis cancer, bladder cancer, kidney cancer, prostate cancer, ureter cancer, renal pelvis carcinoma, vulvar cancer, spinal axis tumor, and central nervous system (CNS) neoplasm.
